(19) **Europäisches Patentamt / European Patent Office / Office européen des brevets**

(11) **EP 2 995 313 A1**

(12) **EUROPEAN PATENT APPLICATION**
published in accordance with Art. 153(4) EPC

(43) Date of publication:
**16.03.2016 Bulletin 2016/11**

(21) Application number: **14794304.7**

(22) Date of filing: **02.05.2014**

(51) Int Cl.:
**A61K 35/50** (2015.01)   **A61K 8/98** (2006.01)
**A61P 9/12** (2006.01)   **A61P 17/18** (2006.01)
**A61P 43/00** (2006.01)   **A61Q 19/00** (2006.01)
**A61Q 19/08** (2006.01)

(86) International application number:
**PCT/JP2014/062154**

(87) International publication number:
**WO 2014/181769 (13.11.2014 Gazette 2014/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.05.2013 JP 2013099670**

(71) Applicant: **Ibiden Co., Ltd.**
**Ogaki-shi, Gifu 503-8604 (JP)**

(72) Inventors:
• **HIROSE, Naohiro**
  **Gifu 503-8604 (JP)**
• **ITO, Yasuaki**
  **Gifu 503-8604 (JP)**

(74) Representative: **Vossius & Partner**
**Patentanwälte Rechtsanwälte mbB**
**Siebertstrasse 3**
**81675 München (DE)**

(54) **PLACENTA EXTRACTS AND METHOD FOR PREPARING SAME**

(57)   The present invention provides: a placenta extract having a free amino acid content of less than or equal to 10 wt% with respect to the total solid content and a peptide content of 70 wt% to 99.5 wt% with respect to the total solid content; a placenta extract having a free amino acid content of less than or equal to 10 wt% with respect to the total solid content and a content of low-molecular peptides of 40 wt% to 99.5 wt% with respect to the total solid content, where the low-molecular peptides have a molecular weight of less than or equal to 3000; and a method of preparing such a placenta extract through subcritical treatment of a raw placenta material and an extraction agent. The placenta extract of the present invention, which is preferably extracted from a raw placenta material through subcritical treatment, has a high content of low-molecular peptides and a low content of free amino acids. Therefore, the placenta extract has remarkably improved antioxidative activity due to SOD-like activity, collagen production accelerating activity, collagenase inhibitory activity, elastase inhibitory activity, and ACE inhibitory activity as compared to that in the related art.

**Description**

Technical Field

The present invention relates to a placenta extract which is obtained by subjecting a placenta, as a raw material, to subcritical treatment, and to a method of preparing a placenta extract in which it is possible to make the placenta extract have a high content of low-molecular peptides.

Background Art

A placenta refers to the placenta of mammals and has recently been used as a health food, a cosmetic material, and a pharmaceutical product due to its excellent functionality. In order for a placenta to exhibit excellent functionality, in general, it has been necessary to degrade a protein in the placenta to a low-molecular peptide, which is excellent in absorbability and functionality, and to extract the low-molecular peptide. In the related art, enzymatic treatment has been exclusively used as a technique for solubilizing a placenta and degrading the placenta so as to have a low molecular weight.

As a specific case thereof, PTL 1 discloses an example in which a melanin formation inhibitor is obtained by adding a yeast extract to a supernatant which is obtained by hydrolyzing a residue which is removed when extracting a high-molecular protein such as a water-soluble protein from the tissue of a human placenta using a proteolytic enzyme.

PTL 2 discloses an example for obtaining a cosmetic composition which contains a water-soluble component which is obtained by hydrolyzing a swine placenta and/or a horse placenta through enzymatic treatment and a polyphenol derivative.

Citation List

Patent Literature

[PTL 1] JP-A-53-142515
[PTL 2] JP-A-2002-212046

Summary of Invention

Technical Problem

The present invention relates to a placenta extract obtained through subcritical treatment of a placenta and preferably through subcritical water treatment of the placenta, and an aqueous solution which contains the extract, and particularly to a placenta extract containing high amounts of low-molecular peptides.

However, in the above-described related art, when performing extraction, it is a matter of course to add an enzyme and it is necessary to use a processing aid in addition to the enzyme. Specifically, in a case of extraction through enzymatic treatment, a pH regulating agent is used as the processing aid in accordance with the type of proteolytic enzyme or other enzyme. When extracting a protein component of a placenta or the like using the processing aid, the concentration of an active component becomes low due to the processing aid, and therefore, the concentration of an active component per extract becomes low.

Furthermore, the processing of extracting a protein component from a placenta in the related art requires post-processing in which a processing aid is further added in order to adjust the pH due to the processing aid. A series of these processes such as the enzymatic treatment and the addition of a processing aid decomposes peptides which are active components that exhibit functionality, and therefore, the number of free amino acids is increased due to the decomposition of the peptides. Due to the increase of the free amino acids, the peptides which are active components in the extract are lost. As a result, in some cases, functionality such as antioxidative activity is not sufficiently exhibited in the obtained extract.

Solution to Problem

The present inventors have conducted extensive studies on a placenta extract, and as a result, they have found characteristics of an extract which can be obtained by subjecting a placenta to subcritical treatment and an aqueous solution which contains the extract, and have found that the above-described placenta extract and the aqueous solution which contains the placenta extract contain low-molecular peptides with a molecular weight of less than or equal to 3000 at a high ratio.

That is, the gist of the present invention relates to the following points:

[1] a placenta extract having a free amino acid content of less than or equal to 10 wt% with respect to the total solid content and a peptide content of 70 wt% to 99.5 wt% with respect to the total solid content;
[2] a placenta extract having a free amino acid content of less than or equal to 10 wt% with respect to the total solid content and a content of low-molecular peptides of 40 wt% to 99.5 wt% with respect to the total solid content, where the low-molecular peptides have a molecular weight of less than or equal to 3000;
[3] a method of preparing a placenta extract through subcritical treatment of a raw placenta material and an extraction agent, in which the placenta extract has a free amino acid content of less than or equal to 10 wt% with respect to the total solid content and a peptide content of 70 wt% to 99.5 wt% with respect to the total solid content; and
[4] a method of preparing a placenta extract through subcritical treatment of a raw placenta material and an extraction agent, in which the placenta extract has a free amino acid content of less than or equal to 10 wt% with respect to the total solid content and a content of low-molecular peptides of 40 wt% to 99.5 wt% with respect to the total solid content, where the low-molecular peptides have a molecular weight of less than or equal to 3000.

Advantageous Effects of Invention

The placenta extract of the present invention, which is preferably extracted from a raw placenta material through subcritical treatment, has a high content of low-molecular peptides and a low content of free amino acids. With such a characteristic composition, antioxidative activity due to super oxide dismutase (SOD)-like activity, collagen production accelerating activity (beautiful skin), collagenase inhibitory activity (beautiful skin), elastase inhibitory activity, angiotensin-converting enzyme (ACE) inhibitory activity (blood pressure elevation inhibitory activity), and the like are remarkably improved as compared to those in the related art.

Brief Description of Drawings

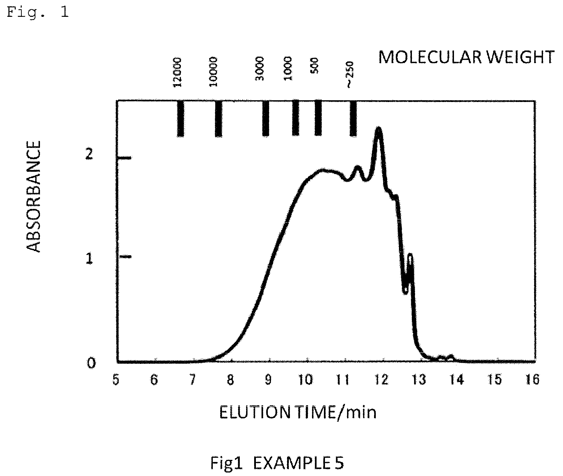

Fig. 1 is a chart showing a molecular weight distribution which was obtained by analyzing a sample which was obtained in Example 5 through high-speed liquid chromatography. The horizontal axis on the lower side of the chart indicates elution time (min), the horizontal axis on the upper side indicates molecular weight corresponding to the elution time, and the longitudinal axis indicates relative absorbance.

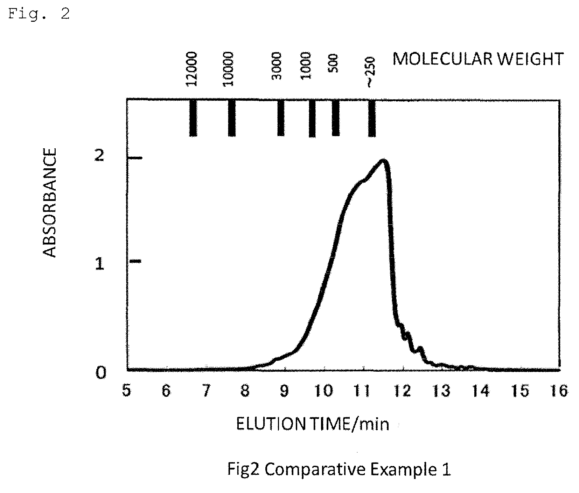

Fig. 2 is a chart showing a molecular weight distribution which was obtained by analyzing a sample which was obtained in Comparative Example 1 through high-speed liquid chromatography. The horizontal axis on the lower side of the chart indicates elution time (min), the horizontal axis on the upper side indicates molecular weight corresponding to the elution time, and the longitudinal axis indicates relative absorbance.

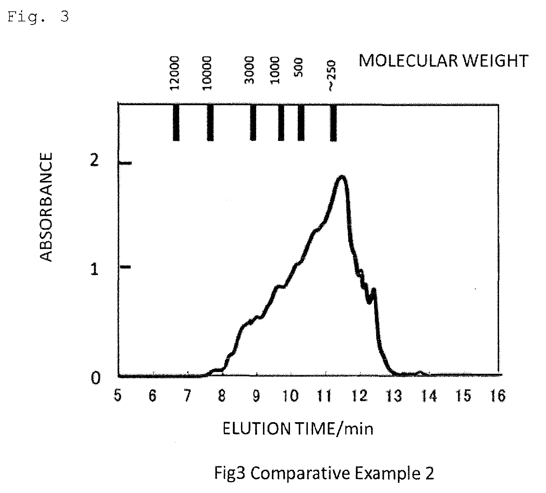

Fig. 3 is a chart showing a molecular weight distribution which was obtained by analyzing a sample which was obtained in Comparative Example 2 through high-speed liquid chromatography. The horizontal axis on the lower side of the chart indicates elution time (min), the horizontal axis on the upper side indicates molecular weight corresponding to the elution time, and the longitudinal axis indicates relative absorbance.

Description of Embodiments

The weight ratio in the present application refers to a weight ratio (wt%) of each component with respect to the total weight of the total solid content of a placenta extract. In a case where the placenta extract contains a liquid component such as moisture, the content of the extract which is in a state where the liquid component has been removed by drying or freeze-drying the placenta extract is regarded as the total solid content of the placenta extract.

In the present specification, the content of free amino acids in the total solid content, the content of peptides in the total solid content, and the content of low-molecular peptides with a molecular weight of less than or equal to 3000 in the total solid content are values which are obtained by acquiring a molecular weight distribution of a dried substance or a freeze-dried substance of a placenta extract using high-speed liquid chromatography or by combining calculation results of the amount of constituent amino acids or the like using an amino acid analyzing method or the like, as described in the following Examples.

In addition, the placenta extract is an extract in which the content of peptides is 70 wt% to 99.5 wt% in the total solid content and the amount of the free amino acids generated can be suppressed such that the content of the free amino acids is less than or equal to 10 wt% in the total solid content. It is possible to obtain such an extract through a production method of the present invention. For this reason, it is possible to obtain a placenta extract in which the content rate of peptides is higher than that of a product in the related art and the content of free amino acids is lower than that of the product in the related art. Accordingly, it is possible to provide a placenta extract with high functionality such as antioxidative activity or collagen production accelerating activity. Here, it is more preferable that the content of peptides in the total solid content is greater than or equal to 75 wt%.

It is possible to obtain a placenta extract by exposing a placenta to a subcritical state without using a processing aid which is used in the enzymatic treatment in the related art.

In addition, the placenta extract is an extract which contains 40 wt% to 99.5 wt% of low-molecular peptides having a molecular weight of less than or equal to 3000 in the total solid content, and in which the amount of the free amino acids generated can be suppressed such that the content of the free amino acids is less than or equal to 10 wt% in the total solid content. It is possible to obtain such an extract through a production method of the present invention. For this reason, it is possible to obtain a placenta extract in which the content rate of low-molecular peptides is higher than that of a product in the related art and the content of free amino acids is lower than that of the product in the related art. Accordingly, it is possible to provide a placenta extract with high functionality such as antioxidative activity or collagen production accelerating activity. Here, it is more preferable that the content of the low-molecular peptides in the total solid content is greater than or equal to 55 wt%.

In the following Examples, molecular weight distributions of a placenta extract which was subjected to subcritical treatment and placenta extracts which were subjected to enzymatic treatment in the related art were measured through high-speed liquid chromatography to be described later. As a result of comparison of the charts thereof which are analysis results, in the placenta extract which was subjected to subcritical treatment, in an area of a molecular weight of less than or equal to 3000, when the height of a maximum peak in a chart was set to greater than or equal to 1, for example, to about 2, 3 or more peaks with relative absorbance (the "relative absorbance" in the present specification means a "relative absorbance" which is defined in this manner) greater than or equal to 0.5 were confirmed through the high-speed liquid chromatography.

The peak referred to herein is a peak showing a result which has a maximum value in the absorbance of the high-speed liquid chromatography and is not a shoulder peak showing a slight change in the absorbance during an increase or decrease of the absorbance.

Specifically, in the placenta extract which was subjected to subcritical treatment, in the area of a molecular weight of less than or equal to 3000, it was confirmed that there were 3 or more peaks with relative absorbance greater than or equal to 0.5, through the high-speed liquid chromatography method to be described later. Moreover, it was confirmed that the peaks were in a molecular weight area of a molecular weight of about 500, a molecular weight area of a molecular weight of about 250, and a molecular weight area of a molecular weight of less than 250.

In the enzymatic method in the related art, one peak was confirmed as a peak with relative absorbance greater than or equal to 0.5 in the area of a molecular weight of less than or equal to 3000, through the high-speed liquid chromatography method to be described later. It was confirmed that the molecular weight of the peak was about 250.

Based on the result, it is possible to generate almost the same amounts of a plurality of peptides which vary in the molecular weight area of a molecular weight of less than or equal to 3000, at the same time in the placenta extract obtained by subjecting a placenta as a raw material to subcritical treatment.

Accordingly, it is estimated that, in the placenta extract which has been subjected to the subcritical treatment, due to the peptides corresponding to 3 or more peaks which have relative absorbance greater than or equal to 0.5 measured through the high-speed chromatography and which vary in the molecular weight area, the amount of peptides acting on functionality such as antioxidative activity or collagen production acceleration is increased, and it is possible to achieve complex functionality due to different actions from each other (for example, an immediate effect and a delayed effect or a functional action and a functionality auxiliary action). By this estimation, it is also estimated that it is possible to obtain an extract which has functionality higher than that of the placenta extract which has been subjected to enzymatic treatment.

In addition, it is estimated that even when the placenta extract which has been subjected to subcritical treatment contains peptides corresponding to a plurality of peaks which have relative absorbance greater than or equal to 0.5 measured through the high-speed chromatography of the placenta extract and which vary in the molecular weight area, the placenta extract has actions equivalent to the above. In other words, it is estimated that the placenta extract which contains peptides corresponding to a plurality of peaks which have relative absorbance greater than or equal to 0.5 measured through the high-speed chromatography and which vary in the molecular weight area is an extract which has functionality higher than the placenta extract which has been subjected to enzymatic treatment.

When the proportion of a free amino acid in the extract increases, the proportion of a peptide in the extract decreases. Therefore, it is difficult for the placenta extract to exhibit the functionality, such as antioxidative activity, collagen production accelerating activity, collagenase inhibitory activity, and blood pressure elevation inhibitory activity. The free amino acid is generated by the decomposition of a peptide by an enzyme or a processing aid which is usually used in order to obtain an extract. As a result, the addition of an enzyme or a processing aid increases the generation of the free amino acid while the enzyme or the processing aid is used for generating a peptide.

The exhibition of functionality such as antioxidative activity or collagen production accelerating activity is inhibited less likely by obtaining a placenta extract of which the content of free amino acids in the total solid content is less than or equal to 10 wit%. The placenta extract which has been subjected to subcritical treatment is obtained without the use of an enzyme or a processing aid in an extraction process, and therefore, free amino acids are hardly generated and it is easy to obtain the placenta extract of which the content of free amino acids in the solid content is less than or equal to 10 wt%.

In contrast, in a placenta extract of which the content of free amino acids in the solid content exceeds 10 wt%, the absorption of a free amino acid in a living body inhibits the absorption of a peptide therein. Accordingly, it is estimated that when there are many free amino acids, the absorbability or the functionality which is originally possessed by a peptide is suppressed, and the functionality is hardly expressed.

When the content of free amino acids is low, the amount of peptides in an extract becomes relatively large. When the amount of peptides in an extract becomes larger, the functionality is more easily exhibited.

In addition, even for the expression of functionality represented by the antioxidative activity, peptides can express functionality which is higher than that of a free amino acid alone since the peptides have a peptide bond with functionality higher than that of the free amino acid due to the structure thereof. Accordingly, even when there are functional peptides, when there is a certain amount or more of free amino acids, the functionality of the peptides is inhibited due to the existence of the amino acids having low functionality.

It is inferred that the concentration of the free amino acids inhibiting the functionality of the peptides generally exceeds 10 wt% of the total solid content in the extract.

In this manner, it is preferable that there is no free amino acid at all in the total solid content or there is a smaller content of free amino acids in the total solid content, in view of exhibiting the function of the placenta extract. Specifically, it is preferable that the content of free amino acids in the solid content is less than or equal to 5 wt%. With this content, the exhibition of the functionality such as antioxidative activity or collagen production accelerating activity is inhibited less likely, and thus, the functionality which is possessed by peptides is easily expressed. Particularly, in the placenta extract which has been subjected to subcritical treatment, free amino acids are hardly generated because an enzyme or a processing aid is not used, and therefore, it is easy to obtain the placenta extract of which the content of free amino acids in the solid content is less than or equal to 5 wt%. It is possible to further suppress the generation of free amino acids by adjusting the conditions such as temperature and time in the subcritical conditions. Therefore, it is easier to obtain the placenta extract of which the content of free amino acids in the solid content is less than or equal to 5 wt%. In addition, there is no problem as a placenta extract even when the content of free amino acids is 0 wt%.

With a placenta extract of which the content of free amino acids in the solid content is less than or equal to 10 wt% and the content of peptides in the solid content is greater than or equal to 70 wt%, the free amino acids in the solid content do not affect the expression of functionality and the peptides easily express functionality. As a result, the functionality such as antioxidative activity or collagen production accelerating activity is exhibited.

Particularly in the placenta extract which has been subjected to subcritical treatment, the generation of free amino acids is suppressed and peptides are easily generated regardless of the molecular weight thereof. As a result, it is possible to obtain a placenta extract of which the content of free amino acids in the solid content is less than or equal to 10 wt% and the total amount of peptides in the solid content is greater than or equal to 70 wt%.

Furthermore, with a placenta extract of which the content of free amino acids in the solid content is less than or equal to 5 wt% and the amount of peptides in the solid content is 70 wt% to 99.5 wt%, the free amino acids in the solid content do not affect the expression of functionality of the peptides derived from the placenta extract and the peptides easily express functionality. As a result, the functionality such as antioxidative activity or collagen production accelerating activity is sufficiently exhibited.

Particularly in the placenta extract which has been subjected to subcritical treatment, the generation of free amino acids is suppressed and peptides are easily generated regardless of the molecular weight thereof. As a result, the placenta extract is a placenta extract of which the content of free amino acids in the solid content is less than or equal to 5 wt% and the content of peptides in the solid content is greater than or equal to 70 wt%. It is possible to obtain such an extract through a production method of the present invention. It is possible to further suppress the generation of free amino acids by adjusting the conditions such as temperature and time in the subcritical conditions. Moreover, peptides are easily generated. Therefore, it is possible to more reliably obtain the placenta extract of which the content of free amino acids in the solid content is less than or equal to 5 wt% and the content of peptides in the solid content is greater than or equal to 70 wt%.

In addition, a placenta extract of which the content of free amino acids in the solid content is less than or equal to 10 wt% and the content of low-molecular peptides with a molecular weight of less than or equal to 3000 in the solid content is 40 wt% to 99.5 wt% can more strongly exhibit the functionality such as antioxidative activity or collagen production accelerating activity.

Particularly in the placenta extract which has been subjected to subcritical treatment, the generation of free amino acids is suppressed and low-molecular peptides with a molecular weight of less than or equal to 3000 are easily generated. As a result, it is possible to obtain a placenta extract of which the content of free amino acids in the solid content is less than or equal to 10 wt% and the content of low-molecular peptides with a molecular weight of less than or equal to 3000 in the solid content is 40 wt% to 99.5 wt%. It is possible to obtain such an extract through a production method of the present invention. Furthermore, the placenta extract which has been subjected to subcritical treatment is an extract which contains low-molecular peptides which have a molecular weight of less than or equal to 3000 and which correspond to 3 or more different peaks in the molecular weight area with relative absorbance greater than or equal to 0.5 measured through the high-speed liquid chromatography to be described later, in the area of a molecular weight of less than or equal to 3000. Moreover, it is estimated that in the extract, the amount of peptides acting on the functionality is increased more than that of a product in the related art, and different actions from each other (for example, an immediate effect and a delayed effect or a functional action and a functionality auxiliary action) can act on complex functionality, and also, the functionality is not inhibited due to the content of free amino acids in the solid content being less than or equal to 10%. By this estimation, it is also estimated that it is possible to obtain an extract which has functionality higher than that of the placenta extract which has been subjected to enzymatic treatment of the product in the related art.

In addition, it is estimated that even when the placenta extract which has been subjected to subcritical treatment contains low-molecular peptides which have a molecular weight of less than or equal to 3000 and which correspond to a plurality of different peaks in the molecular weight area with relative absorbance greater than or equal to 0.5 measured through the high-speed liquid chromatography, in the area of a molecular weight of less than or equal to 3000, the placenta extract has actions equivalent to the above.

Furthermore, with a placenta extract of which the content of free amino acids in the solid content is less than or equal to 5 wt% and the content of low-molecular peptides with a molecular weight of less than or equal to 3000 in the solid content is 55 wt% to 99.5 wt%, the exhibition of the functionality is not inhibited and the functionality such as antioxidative activity or collagen production accelerating activity can be sufficiently exhibited since the content of free amino acids in the extract is low.

Particularly in the placenta extract which has been subjected to subcritical treatment, the generation of free amino acids is suppressed and low-molecular peptides with a molecular weight of less than or equal to 3000 are easily generated. As a result, it is possible to obtain a placenta extract of which the content of free amino acids in the solid content is less than or equal to 5 wt% and the content of low-molecular peptides with a molecular weight of less than or equal to 3000 in the solid content is 55 wt% to 99.5 wt%. Furthermore, the placenta extract which has been subjected to subcritical treatment is an extract which has 3 or more peaks with relative absorbance greater than or equal to 0.5 measured through the high-speed chromatography to be described later, in the area of a molecular weight of less than or equal to 3000. Moreover, it is estimated that the amount of peptides acting on functionality of the extract increases, and it is possible to achieve complex functionality due to different actions from each other (for example, an immediate effect and a delayed effect or a functional action and a functionality auxiliary action), and also, the functionality is not inhibited due to the content of free amino acids in the solid content being less than or equal to 10%. By this estimation, it is also estimated that it is possible to obtain an extract which has functionality higher than that of the placenta extract which has been subjected to enzymatic treatment.

In addition, it is estimated that even when the placenta extract which has been subjected to subcritical treatment is an extract which has a plurality of different peaks with relative absorbance greater than or equal to 0.5 measured through the high-speed liquid chromatography, in the area of a molecular weight of less than or equal to 3000, the placenta extract has actions equivalent to the above.

In addition, it is possible to suppress the generation of free amino acids by adjusting the conditions such as temperature and time in the subcritical conditions. Moreover, peptides with a molecular weight of less than or equal to 3000 are easily generated. Therefore, it is possible to more reliably obtain the placenta extract of which the content of free amino acids in the solid content is less than or equal to 5 wt% and the content of low-molecular peptides with a molecular weight of less than or equal to 3000 in the solid content is 55 wt% to 99.5 wt%.

Here, the extract components in a placenta extract are generally classified into four categories including free amino acids, low-molecular peptides with a molecular weight of less than or equal to 3000, peptides with a molecular weight greater than 3000, and other components.

The free amino acids are 18 kinds of amino acids including arginine, lysine, histidine, phenylalanine, tyrosine, leucine, isoleucine, methionine, valine, alanine, glycine, proline, glutamic acid (including glutamine), serine, threonine, aspartic acid (including asparagine), cystine, and tryptophan which exist independently.

The low-molecular peptides with a molecular weight of less than or equal to 3000 refer to all of the peptides with a molecular weight of less than or equal to 3000 among peptides in which two or more molecules of 18 kinds of amino acids including arginine, lysine, histidine, phenylalanine, tyrosine, leucine, isoleucine, methionine, valine, alanine, glycine, proline, glutamic acid (including glutamine), serine, threonine, aspartic acid (including asparagine), cystine, and tryptophan are bonded. These low-molecular peptides with a molecular weight of less than or equal to 3000 are peptides which have a good absorbability in the human body, and the absorbed component easily exhibits functionality of an extract of a protein derived from a placenta which is represented by antioxidative activity, collagen production accelerating activity, collagenase inhibitory activity, and blood pressure elevation inhibitory activity.

The peptides with a molecular weight greater than 3000 refer to all of the peptides with a molecular weight greater than 3000 among peptides in which two or more molecules of 18 kinds of amino acids including arginine, lysine, histidine, phenylalanine, tyrosine, leucine, isoleucine, methionine, valine, alanine, glycine, proline, glutamic acid (including glutamine), serine, threonine, aspartic acid (including asparagine), cystine, and tryptophan are bonded.

The other components refer to all of the components including inorganic components represented by minerals, lipids, carbohydrates, and the like, other than peptides and amino acids. It is difficult to remove all of the other components through separation and extraction, and therefore, an amount greater than or equal to 0.5 wt% of the other components with respect to the total solid content is contained in a placenta extract.

The constituent amino acids in the present specification refer to a total amount of all of the proteins constituting the placenta and the placenta extract; free amino acids; and amino acids which are contained in peptides. That is, the constituent amino acids refer to all of the proteins; amino acids constituting peptides; and free amino acids which independently exist, regardless of the molecular weight and simple/compound substance.

Specifically, the constituent amino acids in the present application are free amino acids, low-molecular peptides with a molecular weight of less than or equal to 3000, and peptides with a molecular weight greater than 3000, and refer to all of these amino acids. In addition, the amount of constituent amino acids in the present application is a total amount of the amount of free amino acids, the amount of low-molecular peptides with a molecular weight of less than or equal to 3000, and the amount of peptides with a molecular weight greater than 3000.

Furthermore, all of the peptides are all peptides including the low-molecular peptides with a molecular weight of less than or equal to 3000 and the peptides with a molecular weight greater than 3000. In other words, the term all of the peptides refers to all peptides regardless of the molecular weight thereof. The total amount of peptides in the present application is the total amount of the amount of the low-molecular peptides with a molecular weight of less than or equal to 3000 and the amount of the peptides with a molecular weight greater than 3000.

Furthermore, it is desirable that the content of the low-molecular peptides with a molecular weight of less than or equal to 3000 is greater than or equal to 55 wt% by weight ratio in the total solid content of an extract. This is because it is possible to sufficiently exhibit the functionality such as antioxidative activity or collagen production accelerating activity due to a further increased content of peptides.

Regarding the placenta, the species of animals is not limited as long as the placenta is from a mammal. In view of ease of acquiring the placenta, examples of the mammals include humans, pigs, cattle, horses, sheep, and wild boars.

As a raw material of the placenta, greater than or equal to 0.5 wt% of other components such as inorganic components represented by minerals, lipids, carbohydrates, and the like, other than proteins, peptides and free amino acids are included. Accordingly, it is inferred that the upper limit of the content of low-molecular peptides is 99.5 wt% even when all of the peptides can be set to have a molecular weight of less than or equal to 3000 through subcritical treatment. This is a numerical value which has been obtained based on the empirical rule through cumulative experimentation. In addition, it is also inferred that the upper limit of the content of peptides is 99.5 wt%.

In a case where water is used as an extraction agent which is used in subcritical treatment in the present invention, water can be used either in a liquid state or a gaseous state as long as the treatment performed is water treatment at high temperature. That is, either or both of steam and water may be supplied to a treatment tank for subcritical treatment. The temperature of water or steam is desirably greater than or equal to 100°C. As a desirable reaction field, the reaction more easily proceeds in a liquid state than in a gaseous state. Therefore, it is preferable to use water which is forcibly changed into a liquid state in a nearly closed container, that is, in a subcritical state.

The subcritical treatment refers to a treatment in which a predetermined component is extracted from an extraction raw material by bringing a subcritical fluid as an extraction agent which is made to enter a subcritical state under the conditions of a predetermined temperature and pressure, into contact with a raw material (the placenta in the present invention) to be extracted. For example, water enters a state which is neither a liquid nor a gas when the pressure is increased to 22.12 MPa and the temperature is increased to 374.15°C. This point is called the critical point of water, and hot water at a temperature/pressure which is lower than the critical point is called subcritical water. The subcritical water has an excellent component extraction action and hydrolytic action due to a decreased dielectric constant and improvement of ionic products.

More specifically, an extract which is obtained by putting the placenta and water, which is an extraction agent, into a pressure resistant container made of metal, ceramics, or the like to bring both of the placenta and water into contact with each other for a certain period of time or longer in a subcritical state of water (temperature: greater than or equal to 100°C, pressure: greater than or equal to a saturated vapor pressure) by making them enter a state close to a closed state, is regarded as a subcritical treatment extract.

Examples of the extraction agent used in subcritical treatment include ethylene, ethane, propane, carbon dioxide, methanol, ethanol, and a mixture thereof, in addition to water. Among these, water is most preferably used in view of safety.

Next, the treatment conditions when using water as an extraction agent will be described.

The temperature for performing subcritical treatment of the placenta is desirably between 160°C and 200°C. Low-molecular peptides with a molecular weight of less than or equal to 3000 are easily generated by setting the temperature range in this manner and it is possible to obtain the low-molecular peptides which have a molecular weight of less than or equal to 3000 and which correspond to 3 or more different peaks in the molecular weight area with relative absorbance greater than or equal to 0.5 in the molecular weight distribution measurement using the high-speed liquid chromatography to be described later.

There is a tendency that, when the temperature for subcritical treatment is less than 160°C, it is difficult to generate low-molecular peptides with a molecular weight of less than or equal to 3000. There is a tendency that, when the temperature for subcritical treatment exceeds 200°C, the generated low-molecular peptides undergo a further subcritical reaction, and therefore, the generated amount of the low-molecular peptides with a molecular weight of less than or equal to 3000 is decreased.

Furthermore, preferably, the temperature for subcritical treatment is desirably 180°C to 195°C. The temperature is set in this range because low-molecular peptides with a molecular weight of less than or equal to 3000 are easily generated and the generation of free amino acids is easily suppressed to less than or equal to 10 wt%.

It is desirable to perform the subcritical treatment of the placenta at a pressure greater than or equal to a saturated vapor pressure at each temperature (for example, greater than or equal to 0.61 MPa at 160°C and greater than or equal to 1.55 MPa at a temperature greater than or equal to 200°C). The low-molecular peptides with a molecular weight of less than or equal to 3000 are easily generated by setting the pressure in this range. The upper limit of the pressure of the subcritical treatment is not particularly defined, but it is desirable to suppress the pressure thereof to be about 20 MPa to 30 MPa according to the specifications of a high-pressure device used.

The period of time for which the subcritical treatment of the placenta is performed is desirably between 5 minutes to 30 minutes. By setting the time for the treatment in this range, the low-molecular peptides are easily generated and it is possible to obtain low-molecular peptides which have a molecular weight of less than or equal to 3000 and which correspond to 3 or more different peaks in the molecular weight area with relative absorbance greater than or equal to 0.5 measured through the high-speed liquid chromatography to be described later.

There is a tendency that, when the time for subcritical treatment is less than 5 minutes, the low-molecular peptides are hardly generated. There is a tendency that, when the time for subcritical treatment exceeds 30 minutes, the generated low-molecular peptides further undergo excessive decomposition, thereby reducing the amount of the low-molecular peptides generated.

Furthermore, preferably, the treatment time is desirably set to 10 minutes to 30 minutes. The treatment time is set in this range because low-molecular peptides are easily generated and the generation of free amino acids is easily suppressed to less than or equal to 10 wt%.

At this time, as the conditions for hydrolysis through the subcritical treatment of the placenta when water is used as an extraction agent, the treatment is desirably performed at a temperature of 160°C to 200°C, at a pressure of greater than or equal to a saturated vapor pressure at each temperature, and for a period of time of 5 minutes to 30 minutes. This is because low-molecular peptides are easily generated and the generation of free amino acids is suppressed by performing the treatment under these conditions.

Furthermore, preferably, the treatment is desirably performed at a temperature of 180°C to 195°C, at a pressure of greater than or equal to a saturated vapor pressure at each temperature, and for a period of time of 10 minutes to 30 minutes. This is because greater than or equal to 40 wt% of low-molecular peptides in the solid content is easily generated; the generation of free amino acids in the solid content is easily suppressed to less than or equal to 10 wt%; and it is possible to obtain low-molecular peptides which have a molecular weight of less than or equal to 3000 and which correspond to 3 or more different peaks in the molecular weight area with relative absorbance greater than or equal to 0.5 measured through the high-speed liquid chromatography to be described later, by setting the ranges in this manner.

It is preferable to subject a subcritically treated substance which is obtained through the subcritical treatment subsequently to solid-liquid separation to collect a liquid. It is possible to use the collected liquid as an aqueous solution which contains a placenta extract as it is. In addition, it is possible to obtain a solid placenta extract by drying the collected liquid. Needless to say, it is possible to obtain an aqueous solution which contains a placenta extract by dissolving the solid placenta extract into an aqueous solution again.

A general solid-liquid separation method can be used in order to obtain a liquid portion by subjecting a treated substance which is obtained through the subcritical treatment to solid-liquid separation. Specifically, the separation can be performed through any of filtration using filter paper, centrifugation, decantation, screw press, roller press, rotary drum screen, belt screen, vibrating screen, multi-plate vibration filter, vacuum dehydration, pressure dehydration, belt press, centrifugal concentration dehydration, and multiple disc dehydration.

It is possible to obtain a solid placenta extract by drying the liquid which is obtained after the solid-liquid separation. It is possible to use a general drying method for this method. It is possible to perform heat transfer drying, such as box-type drying or spray drying, and internal heat generation drying such as microwave drying, which are heating systems; and freeze-drying, vacuum drying, suction drying, press drying, and ultrasonic drying, which are non-heating systems, not to mention natural standing. As a matter of course, drying using a simple oven and a thermostat tank may be generally allowed.

The placenta extract which is obtained through the subcritical treatment can be obtained as a low-molecular peptide extract regardless of a liquid content or a solid content.

Examples

Hereinafter, the present invention will be more specifically described using examples, but is not limited to these examples.

(Preparation of Placenta Extract)

Example 1

200 g of a swine placenta (Tokyo Shibaura Zouki Co., Ltd.; hereinafter, referred to as the swine placenta) and 200 g of distilled water were put into a pressure resistant container with a 2L capacity, to perform subcritical treatment under the conditions of the treatment temperature of 180°C, the treatment pressure of 1.0 MPa, and the treatment time of 10 minutes.

After completion of the subcritical treatment, a treated substance within the pressure resistant container was collected and was filtered using filter paper made of cellulose (hole diameter: 1 $\mu$m; and trade name: 5C manufactured by Advantec) to collect a filtered liquid. A placenta extract was obtained by performing freeze-drying on the filtered liquid. Hereinafter, the extract will be called a sample.

Example 2

The preparation was performed under the same conditions as those in Example 1, except that the subcritical treatment was performed under the conditions of the treatment temperature of 180°C, the treatment pressure of 1.0 MPa, and the treatment time of 30 minutes.

Example 3

The preparation was performed under the same conditions as those in Example 1, except that the subcritical treatment was performed under the conditions of the treatment temperature of 188°C, the treatment pressure of 1.2 MPa, and the treatment time of 20 minutes.

Example 4

The preparation was performed under the same conditions as those in Example 1, except that the subcritical treatment was performed under the conditions of the treatment temperature of 195°C, the treatment pressure of 1.6 MPa, and the treatment time of 10 minutes.

Example 5

The preparation was performed under the same conditions as those in Example 1, except that the subcritical treatment was performed under the conditions of the treatment temperature of 195°C, the treatment pressure of 1.6 MPa, and the treatment time of 30 minutes.

Comparative Example 1

(Conditions for Preparing Extract through Enzymatic Treatment)

200 g of the swine placenta, 50 ml of water, 4 ml of Alcalase (manufactured by Novozymes) which is a protein hydrolase, and 2 ml of 25 wt% sodium hydroxide were added and reacted together for 3 hours at 60°C. Then, the reactant was treated for 1 hour at 90°C and a filtered liquid was collected by filtering the treated liquid in which the enzyme was deactivated using filter paper made of cellulose (hole diameter: 1 $\mu$m; and trade name: 5C manufactured by Advantec). The filtered liquid was subjected to freeze-drying and was powdered.

Comparative Example 2

A commercial product A of a powder placenta extract which was subjected to enzymatic treatment was used as an enzyme-treated product of a swine placenta. The commercial product A is a product in which a swine placenta was subjected to protease treatment using papain under the conditions of 2 hours and 35°C and purified.

(Evaluation Method of Molecular Weight Distribution)

The evaluation of a molecular weight distribution was performed using samples of Examples 1 to 5 and Comparative Examples 1 and 2.

The analysis method was such that a 4 wt% solution was prepared by dissolving placenta powder into distilled water, the dissolved powder was filtered through a 0.45 $\mu$m membrane filter, and measurement was performed through high-speed liquid chromatography (HP 1100 series manufactured by Agilent Technologies Japan, Ltd.).

A calibration curve was created similarly through the measurement by standard reagents for molecular weight distribution measurement including Cytochrome C (Wako Pure Chemical Industries, Ltd.) with a molecular weight of 12,500, Aprotinin (CALBOCHEM) with a molecular weight of 6,512, Bacitracin (Dr. Ehrenstorfer) with a molecular weight of 1,423, Angiotensin II (Peptide Institute, Inc.) with a molecular weight of 1,026, Gly-Gly-Tyr-Arg (Peptide Institute, Inc.) with a molecular weight of 451, and Gly-Gly-Gly (Peptide Institute, Inc.) with a molecular weight of 189, using columns (manufactured by Tosoh Corporation, product names: TSK guard column SWXL (6.0 mm I.D. x 40 mm) and TSK gel G2000 SWXL (7.8 mm I.D. x 300 mm)) under the analysis conditions of an eluent of 0.1 w/v% TFA in MeCN/H$_2$O=45/55, the column temperature of 35°C, the flow velocity of 1.0 mL/min, the detection UV of 220 nm, the introduction amount of 20 $\mu$L, and the analysis time of 30 min; the position of a molecular weight with respect to elution time was determined; the areas of peaks in molecular weight distribution and molecular weight fraction ranges were obtained; and the molecular weight proportion was obtained. The result is shown in Table 1.

The upper limits of the molecular weights which were obtained this time in all of the samples were less than or equal to an extra exclusion molecular weight (about 100,000) of the columns and a maximum molecular weight which was calculated from the time at which the elution started was about 20,000. Accordingly, it was found that all of the molecular weights of proteins contained in raw materials in the above-described examples and comparative examples were less than or equal to about 100,000 and the proteins were contained in the samples by being converted into a peptide form with a molecular weight of less than or equal to about 20,000.

[Table 1]

Table 1 Examples and Comparative Examples Molecular Weight Distribution Proportion

| Item | Molecular weight proportion (proportion in solid content) | |
|---|---|---|
| | Molecular weight $\leq$ 3000 | Molecular weight > 3000 |
| Example 1 | 54.8% | 45.2% |
| Example 2 | 78.4% | 21.6% |
| Example 3 | 80.8% | 19.2% |
| Example 4 | 74.8% | 25.3% |
| Example 5 | 90.6% | 9.4% |
| Comparative Example 1 | 98.0% | 1.9% |
| Comparative Example 2 | 91.5% | 8.5% |

Figs. 1 to 3 are charts showing molecular weight distributions which were obtained by analyzing the samples which were obtained in Example 5 and Comparative Examples 1 and 2 through high-speed liquid chromatography. The horizontal axes on the lower sides of the charts indicate elution time (min), the horizontal axes on the upper sides indicate molecular weight corresponding to the elution time, and the longitudinal axes indicate relative absorbance. The molecular weight distribution observed in Example 5 has rough peaks in three areas including a molecular weight area of a molecular weight of about 500, a molecular weight area of a molecular weight of about 250, and a molecular weight area of a molecular weight of less than 250 (it is difficult to accurately measure the molecular weight since it is difficult to estimate the molecular weight from the behavior of the calibration curve when the molecular weight is less than or equal to about 300, due to characteristics of the columns used this time, but the molecular weight is expressed as less than 250 from the above-described experiment conditions).

In contrast, in Comparative Examples 1 and 2, the observed molecular weight distributions each have only one peak in a molecular weight area near the molecular weight of 250. The tendency of having the three peaks in Example 5 is also observed in other Examples 1 to 4, and under the subcritical conditions, it is possible to obtain an extract which contains peptides having a molecular weight distribution which cannot be obtained through the existing enzymatic treatment as well as peptides which are obtained through the existing enzymatic treatment.

(Measurement of Amount of Free Amino Acids and Amount of Constituent Amino Acids)

Constituent amino acids and free amino acids of the samples in Examples 1 to 5 and Comparative Examples 1 and 2 were analyzed (the analysis was requested to be performed at Food Analysis Technology Center SUNATEC).

After subjecting each of the samples to performic acid oxidation treatment, hydrochloric acid hydrolysis was performed. A total of 18 kinds of amino acids including arginine, lysine, histidine, phenylalanine, tyrosine, leucine, isoleucine, methionine, valine, alanine, glycine, proline, glutamic acid (including glutamine), serine, threonine, aspartic acid (including asparagine), cystine, and tryptophan was measured. The analysis except for tryptophan was measured by an amino acid automatic analysis device and tryptophan was measured by a high-speed chromatography method.

The free amino acids were measured by performing similar analysis of the samples with respect to each amino acid as it was. With respect to the measurement, the amounts of 18 kinds of free amino acids and the amounts of constituent amino acids were respectively added up and the amount of free amino acids and the amount of constituent amino acids in the solid content of an extract were obtained based on the result. Furthermore, the amount of each of the peptides (peptides, peptides with a molecular weight of less than or equal to 3000, and peptides with a molecular weight greater than 3000) was obtained based on the result of Table 1. The result is shown in Table 2.

Amount of peptides = amount of constituent amino acids – amount of free amino acids

Amount of peptides with a molecular weight of less than or equal to 3000 = (amount of constituent amino acids) x (distribution proportion of molecular weight of less than or equal to 3000) – amount of free amino acids

Amount of peptides with a molecular weight greater than 3000 = (amount of peptides – amount of peptides with a molecular weight of less than or equal to 3000)

[Table 2]

Table 2 Amount of Constituent Amino Acids, Amount of Free Amino Acids, and Amount of Peptides (Wt%) in Solid Content of Extract

| Item | Amount of constituent amino acids | Amount of free amino acids | Amount of peptides | | |
|---|---|---|---|---|---|
| | | | Peptides | Peptides with molecular weight of less than or equal to 3000 | Peptides with molecular weight greater than 3000 |
| Example 1 | 79.6 | 1.4 | 78.2 | 42.2 | 36.0 |
| Example 2 | 79.0 | 2.4 | 76.6 | 59.5 | 17.1 |
| Example 3 | 79.5 | 2.3 | 77.2 | 61.9 | 15.2 |

(continued)

| Item | Amount of constituent amino acids | Amount of free amino acids | Amount of peptides | | |
| --- | --- | --- | --- | --- | --- |
| | | | Peptides | Peptides with molecular weight of less than or equal to 3000 | Peptides with molecular weight greater than 3000 |
| Example 4 | 80.7 | 2.0 | 78.7 | 58.4 | 20.3 |
| Example 5 | 73.7 | 3.3 | 70.4 | 63.5 | 6.9 |
| Comparative Example 1 | 73.8 | 15.6 | 58.2 | 56.7 | 1.4 |
| Comparative Example 2 | 70.7 | 16.0 | 54.7 | 48.7 | 5.9 |

(Evaluation of Functionality: Evaluation of Antioxidative Activity through Measurement of SOD-like Activity)

The measurement of SOD-like action activity was performed on the samples in Examples 1 to 5 and Comparative Examples 1 and 2.

An SOD measurement kit (manufactured by Dojindo Laboratories) was used for measuring the SOD-like action activity. An inhibition rate was obtained from the measurement to calculate the concentration (IC50 value) of additives at which the SOD-like activity was inhibited by 50%. The result is shown in Table 3.

```
Inhibition rate (%)

= 1 - (Absorbance of sample solution after enzyme reaction

- absorbance of sample solution when buffer solution was added  instead  of  enzyme) / (absorbance  of  blank  after enzyme reaction - absorbance of blank when buffer solution was added instead of enzyme) x 100.
```

[Table 3]

Table 3 Antioxidative Activity (SOD-like Activity Evaluation) in Examples 1 to 5 and Comparative Examples 1 and 2

| Item | IC50 Value |
| --- | --- |
| Example 1 | 9 mg/ml |
| Example 2 | 6 mg/ml |
| Example 3 | 6 mg/ml |
| Example 4 | 6 mg/ml |
| Example 5 | 3 mg/ml |
| Comparative Example 1 | 27 mg/ml |
| Comparative Example 2 | 96 mg/ml |

It was found from Table 3 that the antioxidative activity of the products (Examples 1 to 5) of the present invention is remarkably higher than that of the products (Comparative Examples 1 and 2) in the related art.

(Evaluation of Functionality: Evaluation of Antioxidative Activity through ORAC Evaluation)

As a test for evaluating antioxidative activity, an ORAC evaluation was performed. That is, reactive oxygen which is generated from AAPH (2,2'-Azobis[2-amidinopropane]dihydrochloride) as a radical generating agent decomposes fluorescein as a fluorescent substance. The area of a decreasing curve was calculated by measuring the fluorescence intensity over time, and the calculated area thereof was compared with that of a standard substance.

When there is an antioxidant substance in the above-described samples or the like, the decomposition rate of fluorescein is reduced because reactive oxygen is eliminated. The reduction degree of the decomposition rate of fluorescein is compared with that of the decomposition rate of fluorescein in the presence of Trolox (6-hydroxy-2,5,7,8-tetrametylchroman-2-carboxylic acid) which is water soluble vitamin E and which is a standard substance of the antioxidant substance, and is calculated as Trolox Equivalent (TE: amount equivalent to water soluble vitamin E) as a unit. When the calculated numerical value is large, the antioxidative activity of the above-described treatment solutions or the like becomes large.

The samples (20 $\mu$l) in Example 5 and Comparative Example 2 which were re-dissolved in a liquid mixture (1 ml) of acetone : water : acetic acid (70: 29.5: 0.5), a diluted solution (20 $\mu$l) in which a 100 $\mu$M Trolox aqueous solution was timely diluted, and a blank (acetone: water: acetic acid (70: 29.5: 0.5) mixture (20 $\mu$l) were dispensed into microplates, and 94.4 nM fluorescein sodium salt (200 $\mu$l) was added thereto, and the mixtures were shaken within a thermostat in the air for 30 minutes at 37°C. 31.7 mM AAPH (75 $\mu$l) was added thereto, and the fluorescence intensity was measured up to 90 minutes after the addition of AAPH every 2 minutes at an excitation wavelength Ex of 485 nm and a fluorescent wavelength Em of 538 nm, using TECMAN Infinite F200 as a fluorescent plate reader.

The antioxidative value was calculated as TE ($\mu$mol TE/g) per amount of an extract. The results of the antioxidative value of each sample are shown in Table 4.

[Table 4]

Table 4 Antioxidative Activity (ORAC Evaluation) in Example 5 and Comparative Example 2

| Item | ORAC Value |
|---|---|
| Example 5 | 569 $\mu$mol TE/g |
| Comparative Example 2 | 274 $\mu$mol TE/g |

It was found from Table 4 that the antioxidative activity of the product (Example 5) of the present invention is remarkably higher than that of the product (Comparative Example 2) in the related art.

(Evaluation of Functionality: Effect for Beautiful Skin through Evaluation of Collagen Production Accelerating Activity)

The evaluation of collagen production accelerating activity was performed using the samples in Example 5 and Comparative Example 2.

Normal human fibroblasts (NB1RGB) which had been stored in liquid nitrogen were defrosted and were cultured in a Doulbecco's modified Eagle's Medium (DMEM medium) containing 5 wt% fetal bovine serum (FBS) until the fibroblasts became sufficiently larger in number to be supplied to a test.

The cultured samples were diluted by 3 times in the 0.5 wt% FBS-containing DMEM medium and test solutions at a total of 4 concentrations were prepared. After collecting the cells of which the number became sufficiently larger to be supplied to a test through trypsin treatment, the treated cells were sowed in each well of a 96-well microplate to perform pre-culturing for 24 hours. Then, the 5% FBS-containing DMEM medium was removed from the 96-well microplate, the medium was replaced with media containing the samples at each concentration, and the culturing was further continued for 48 hours. The amount of collagen generated by each test sample was calculated in accordance with a protocol of a human collagen (I type) Enzyme-Linked ImmunoSorbent Assay (ELISA) method.

In addition, after removing the medium from the 96-well microplate in which the cells were cultured, the cell survival rate was measured through MTT (3-(4,5-di-methylthiazol-2-yl)-2,5-diphenyltetrazolium bromide, yellow tetrazole) assay to calculate the collagen concentration ($\mu$g/ml) per 100% cell survival rate. The results are shown in Table 5.

[Table 5]

Table 5 Evaluation of Collagen Production Accelerating Activity: Collagen Concentration ($\mu$g/ml) per 100% Cell Survival Rate in Example 5 and Comparative Example 2

| Addition concentration | 0.05 wt/vol% | 0.15 wt/vol% | 0.44 wt/vol% | 1.33 wt/vol% |
|---|---|---|---|---|
| Example 5 | 1.3 | 2.0 | 3.1 | 3.3 |

(continued)

| Addition concentration | 0.05 wt/vol% | 0.15 wt/vol% | 0.44 wt/vol% | 1.33 wt/vol% |
|---|---|---|---|---|
| Comparative Example 2 | 0.6 | 0.7 | 1.1 | 2.6 |

It was found from Table 5 that the collagen production accelerating activity of the product (Example 5) of the present invention is remarkably higher than that of the product (Comparative Example 2) in the related art. Based on this point, it is suggested that the product of the present invention has an effect of keeping the skin of a human beautiful.

(Evaluation of Functionality: Effect for Beautiful Skin through Evaluation of Collagenase Inhibitory Activity)

The evaluation of collagenase inhibitory activity was performed using the samples in Example 5 and Comparative Example 2.

A Pz-peptide solution (manufactured by BACHEM Fenichemikalien AG, trade name: Pz-peptide) and a collagenase solution (manufactured by SIGMA Corporation, trade name: collagenase Type IV) were added to diluted solutions of the respective samples (two samples with a sample addition concentration of 4 wt/vol% and with a sample addition concentration of 20 wt/vol% were prepared), and the solutions were reacted at 37°C. Then, the reaction was stopped by adding a 25 mmol/L citric acid solution thereto, and ethyl acetate was added thereto to perform centrifugation. The absorbance of an ethyl acetate layer was measured at a wavelength of 320 nm and the collagenase activity inhibition rate (%) was calculated. The results are shown in Table 6.

```
Collagenase Activity Inhibition Rate (%)
= 1 - (Absorbance of sample solution after reaction -
absorbance of sample solution when purified water was
added instead of enzyme) / (absorbance of blank after
reaction - absorbance of blank when purified water was
added instead of enzyme) x 100
```

[Table 6]

Table 6 Collagenase Inhibitory Activity Rate in Example 5 and Comparative Example 2

| Addition concentration | 4 wt/vol% | 20 wt/vol% |
|---|---|---|
| Example 5 | 23.9% | 85.1% |
| Comparative Example 2 | 0.0% | 41.8% |

It was found from Table 6 that the collagenase activity inhibitory capacity of the product (Example 5) of the present invention is remarkably higher than that of the product (Comparative Example 2) in the related art. Based on this point, it is suggested that the product of the present invention has an effect of keeping the skin of a human beautiful.

(Evaluation of Functionality: Blood Pressure Elevation Inhibitory Effect through Evaluation of ACE Inhibitory Activity)

The evaluation of ACE inhibitory activity was performed using the samples in Example 5 and Comparative Example 2.

After extracting 1.0 g of each sample using 20 ml of a 50 vol% ethanol solution, the extracted sample was diluted 10 times in a 0.1 mol/l HEPES (4-(2-hydroxyethyl)-1-piperazineethanesulfonic acid) buffer solution (pH 8.3) to prepare a test solution. 25 μl of the 0.1 mol/l HEPES buffer solution (pH 8.3) (untreated section) or a test solution was added to 96-well microplate, 25 μl of a 20 mU/ml ACE solution was added thereto, and the mixture was kept warm for 5 minutes at 37°C. 25 μl of 8 mmol/l Hip-His-Leu(Hippuryl-L-histidyl-L-Leucine) solution was added thereto and the mixture was reacted for 30 minutes at 37°C.

Thereafter, the reaction was stopped by adding 25 μl of a 0.1 mol/l sodium hydroxide solution, 25 μl of 1 wt% o-Phthalaldehyde (OPA) solution was added thereto, and the mixture was allowed to stand for 20 minutes at room temperature. 25 μl of 0.1 mol/l hydrochloric acid was added thereto and the mixture was allowed to stand for 10 minutes at room temperature. Then, the fluorescence intensity was measured at an excitation wavelength of 355 nm and a fluorescent wavelength of 460 nm, using a microplate reader (SpectraMax M2e manufactured by Molecular Device Japan). The test of the blank was performed similarly to the test above using a phosphate buffer solution (PBS) instead of the 20 mU/ml ACE solution. The inhibition rate was obtained therefrom and an IC50 value was calculated as a sample concentration at which the ACE activity was inhibited by 50%. The results are shown in Table 7.

```
ACE activity inhibition rate (%)

= 1 - (Fluorescence intensity of sample solution after reaction - fluorescence intensity of sample solution when PBS was added instead of enzyme) / (fluorescence intensity

of blank after reaction - fluorescence intensity of blank when PBS was added instead of enzyme) x 100
```

[Table 7]

Table 7 IC50 Value of Evaluation of ACE Inhibitory Activity in Example 5 and Comparative Example 2

| Item | IC50 Value |
| --- | --- |
| Example 5 | 0.21 mg/ml |
| Comparative Example 2 | 0.48 mg/ml |

It was found from Table 7 that the ACE activity inhibitory capacity of the product (Example 5) of the present invention is remarkably higher than that of the product (Comparative Example 2) in the related art. Based on this point, it is suggested that the product of the present invention has a blood pressure elevation inhibitory effect.

As described above, it was shown that a placenta extract which is obtained through subcritical treatment contains 0.1 wt% to 10 wt% of free amino acids and 40 wt% to 99.5 wt% of low-molecular peptides with a molecular weight of less than or equal to 3000, as main components, and that the functionality of the placenta extract is high.

(Evaluation of Functionality: Effect for Beautiful Skin through Evaluation of Elastase Inhibitory Activity)

Elastase is a kind of protease capable of decomposing elastin, and a decrease in elastin is regarded as a cause of generation of wrinkles on the skin. Accordingly, it can be expected that the component which inhibits the activity of elastase has an effect of keeping the skin of a human beautiful.

Thus, the evaluation of elastase inhibitory activity was performed using the samples of Example 5 and Comparative Examples 1 and 2.

100 μL of a 1 mM N-Succinyl-Ala-Ala-p-nitroanilide (manufactured by Biochem AG) / 50 mM Tris-HCl (tris(hydroxymethyl)aminomethane) buffer solution (pH 8.8), and a 0.5 units/ml swine-derived pancreas elastase (manufactured by Wako Pure Chemical Industries, Ltd.) / 50 mM Tris-HCl buffer solution (pH 8.8) as an enzyme solution were added to 50 μl of a diluted solution of each sample, and the mixture was reacted for 15 minutes at 37°C. Then, the absorbance of the reaction solution was measured at a wavelength of 405 nm, the elastase activity inhibition rate (%) was calculated, and an IC50 value was calculated as a sample concentration at which the elastase activity was inhibited by 50%. The results are shown in Table 8.

```
Elastase Activity Inhibition Rate (%)

= 1 - (Absorbance of sample solution after reaction -

absorbance of sample solution when purified water was added instead of enzyme) / (absorbance of blank after reaction - absorbance of blank when purified water was added instead of enzyme) x 100
```

[Table 8]

Table 8 Evaluation of Elastase Inhibitory Activity

| Item | IC50 Value |
| --- | --- |
| Example 5 | 6 mg/ml |
| Comparative Example 1 | 32 mg/ml |
| Comparative Example 2 | 94 mg/ml |

It was found from Table 8 that the elastase activity inhibitory capacity of the product (Example 5) of the present invention is remarkably higher than that of the products (Comparative Examples 1 and 2) in the related art. Based on this point, it is suggested that the product of the present invention has an effect of keeping the skin of a human beautiful.

Industrial Applicability

The placenta extract of the present invention has functions such as antioxidative activity, collagen production accelerating activity, collagenase activity inhibitory capacity, and ACE inhibitory activity. Therefore, it is possible to use the placenta extract in a raw material for pharmaceutical products, a formulated component of cosmetics, and food products such as a drink or a supplement.

**Claims**

1.  A placenta extract having
    a free amino acid content of less than or equal to 10 wt% with respect to the total solid content, and
    a peptide content of 70 wt% to 99.5 wt% with respect to the total solid content.

2.  A placenta extract having
    a free amino acid content of less than or equal to 10 wt% with respect to the total solid content, and
    a content of low-molecular peptides of 40 wt% to 99.5 wt% with respect to the total solid content, wherein the low-molecular peptides has a molecular weight of less than or equal to 3000.

3.  The placenta extract according to claim 1 or 2 which is extracted from a raw placenta material through subcritical treatment.

4.  The placenta extract according to claim 3,
    wherein the temperature for the subcritical treatment is 160°C to 200°C.

5.  The placenta extract according to claim 3 or 4,
    wherein the period of the subcritical treatment is 5 minutes to 30 minutes.

**6.** The placenta extract according to any one of claims 3 to 5, further comprising:

> a step of collecting a liquid by subsequently subjecting the treated substance obtained through the subcritical treatment to solid-liquid separation treatment.

**7.** An aqueous solution which contains the placenta extract according to any one of claims 1 to 6.

**8.** A method of preparing a placenta extract through subcritical treatment of a raw placenta material and an extraction agent,
wherein the placenta extract has a free amino acid content of less than or equal to 10 wt% with respect to the total solid content, and
a peptide content of 70 wt% to 99.5 wt% with respect to the total solid content.

**9.** A method of preparing a placenta extract through subcritical treatment of a raw placenta material and an extraction agent,
wherein the placenta extract has a free amino acid content of less than or equal to 10 wt% with respect to the total solid content, and
a content of low-molecular peptides of 40 wt% to 99.5 5 wt% with respect to the total solid content, wherein the low-molecular peptides has a molecular weight of less than or equal to 3000.

**10.** The method of preparing a placenta extract according to claim 8 or 9,
wherein the temperature for the subcritical treatment is 160°C to 200°C.

**11.** The method of preparing a placenta extract according to any one of claims 8 to 10,
wherein the period of the subcritical treatment is 5 minutes to 30 minutes.

**12.** The method of preparing a placenta extract according to any one of claims 8 to 11,
wherein the extraction agent is one or more kinds selected from the group consisting of water, ethylene, ethane, propane, carbon dioxide, methanol, and ethanol.

**13.** The method of preparing a placenta extract according to any one of claims 8 to 11, further comprising:

> a step of collecting a liquid by subsequently subjecting the treated substance obtained through the subcritical treatment to solid-liquid separation treatment.

Fig. 1

Fig1 EXAMPLE 5

Fig. 2

Fig2 Comparative Example 1

Fig. 3

Fig3 Comparative Example 2

<div align="center">

**INTERNATIONAL SEARCH REPORT**

</div>

| | International application No. |
|---|---|
| | PCT/JP2014/062154 |

**A. CLASSIFICATION OF SUBJECT MATTER**
*A61K35/50*(2006.01)i, *A61K8/98*(2006.01)i, *A61P9/12*(2006.01)i, *A61P17/18*(2006.01)i, *A61P43/00*(2006.01)i, *A61Q19/00*(2006.01)i, *A61Q19/08*(2006.01)i

According to International Patent Classification (IPC) or to both national classification and IPC

**B. FIELDS SEARCHED**

Minimum documentation searched (classification system followed by classification symbols)
A61K35/50, A61K8/98, A61P9/12, A61P17/18, A61P43/00, A61Q19/00, A61Q19/08

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)
CAplus/MEDLINE/EMBASE/BIOSIS(STN), JSTPlus/JMEDPlus/JST7580(JDreamIII), CiNii, Thomson Innovation, G-Search

**C. DOCUMENTS CONSIDERED TO BE RELEVANT**

| Category* | Citation of document, with indication, where appropriate, of the relevant passages | Relevant to claim No. |
|---|---|---|
| X/Y | JP 2006-149290 A (Masateru EGASHIRA), 15 June 2006 (15.06.2006), abstract; claims; paragraph [0003] (Family: none) | 1/1-13 |
| Y | JP 58-072521 A (Kiichiro OZAKI), 30 April 1983 (30.04.1983), page 5, lower right column (Family: none) | 1-13 |
| Y | JP 2007-135488 A (Nippon Meat Packers, Inc.), 07 June 2007 (07.06.2007), abstract; claims; paragraphs [0011], [0013] (Family: none) | 1-13 |

☒ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 July, 2014 (28.07.14) | 05 August, 2014 (05.08.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2014/062154

C (Continuation).   DOCUMENTS CONSIDERED TO BE RELEVANT

| Category* | Citation of document, with indication, where appropriate, of the relevant passages | Relevant to claim No. |
|---|---|---|
| Y | PLAZA, M. et al., Facts about the formation of new antioxidants in natural samples after subcritical water extraction, Food Research International, 2010, Vol.43, No.10, pp.2341-2348 Abstract | 1-13 |
| Y | JP 2009-057325 A  (Kaneka Corp.), 19 March 2009 (19.03.2009), paragraph [0025] (Family: none) | 1-13 |
| P,Y | LEE, Mi-Yeon, et al., Effects of High Pressure/ High Temperature Processing on the Recovery and Characteristics of Porcine Placenta Hydrolysates, Korean Journal for Food Science of Animal Resources, 2013, Vol. 33, No.4, pp. 474-480 | 1-13 |
| P,Y | HAN, JeungHi, et al., Skin Permeability of Porcine Placenta Extracts and Its Physiological Activities, Korean Journal for Food Science of Animal Resources, 2013, Vol. 33, No.3, pp.356-362 | 1-13 |

Form PCT/ISA/210 (continuation of second sheet) (July 2009)

**EP 2 995 313 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 53142515 A
- JP 2002212046 A